# EUROPÄISCHE PATENTSCHRIFT

(11) **EP 2 614 044 B1**
(45) Veröffentlichungstag und Bekanntmachung des Hinweises auf die Patenterteilung: **08.10.2014**
(21) Anmeldenummer: 11751819.1
(22) Anmeldetag: 18.08.2011
(51) Int. Cl.: C07C 209/26

(54) **VERFAHREN ZUR HERSTELLUNG PRIMÄRER ALIPHATISCHER AMINE AUS ALDEHYDEN**
METHOD FOR PRODUCING PRIMARY ALIPHATIC AMINES FROM ALDEHYDES
PROCÉDÉ DE PRÉPARATION D'AMINES ALIPHATIQUES PRIMAIRES À PARTIR D'ALDÉHYDES

(30) Priorität: 11.09.2010 DE 102010045142
(43) Veröffentlichungstag der Anmeldung: 17.07.2013
(73) Patentinhaber: OXEA GmbH, 46147 Oberhausen (DE)
(72) Erfinder: SCHALAPSKI, Kurt, 46147 Oberhausen (DE); NOWOTNY, Norman, 45276 Essen (DE); EISENACHER, Matthias, 46485 Wesel (DE); BERMANN, Dirk, 45475 Mülheim (DE); KREICKMANN, Thorsten, 46149 Oberhausen (DE); HEYMANNS, Peter, 45147 Essen (DE); STRUTZ, Heinz, 47445 Moers (DE)
(86) Internationale Anmeldenummer: PCT/EP2011/004156
(87) Internationale Veröffentlichungsnummer: WO 2012/031672

(56) Entgegenhaltungen:
- DE-A1- 19 935 448

## Beschreibung

Aliphatische Amine sind wichtige organische Zwischenprodukte, die in großem industriellem Maßstab hergestellt werden. Beispielsweise werden sie für die Herstellung von Agrochemikalien oder Farbstoffen weiterverarbeitet oder sie dienen als Zusatz in oberflächenaktiven Formulierungen, als Korrosionsinhibitor in Schmiermitteln oder als Hilfsstoffe für die Papier, Textil und Kautschukindustrie.

Es ist bekannt, primäre aliphatische Amine aus Aldehyden und Ammoniak mit Wasserstoff an einem Katalysator herzustellen. Man bezeichnet diese Reaktion auch als reduktive Aminierung. Die Aminbildung kann durch folgende Reaktionsstufen beschrieben werden:

R-C(=O)H + NH₃ → R-C(=NH)H + H₂O (1)

R-C(=NH)H + H₂ → R-CH₂-NH₂ (2)

In der ersten Reaktionsstufe entsteht zunächst unter Wasserabspaltung ein Imin, das anschließend in einer zweiten Reaktionsstufe katalytisch hydriert wird.

Bei der Reaktionsführung treten jedoch unerwünschte Nebenreaktionen auf. Zum einen kann es durch die Direkthydrierung der Einsatzaldehyde zur Alkoholbildung kommen. Auch kann der Einsatzaldehyd in dem basischen Medium einer Aldolkondensation unterliegen und bereits gebildetes primäres Amin kann mit dem Einsatzaldehyd über die Azomethinzwischenstufe zu einem sekundären Amin abreagieren, das dann noch analog zu einem tertiären Amin weiterreagieren kann. Auch enthalten die Aldolkondensationsprodukte reaktive Gruppen, die mit den stickstoffhaltigen Verbindungen höhersiedende Kondensationsprodukte bilden können. Um die Selektivität in Richtung der primären aliphatischen Amine zu verbessern und um die Bildung von hochsiedenden Nebenprodukten zurückzudrängen, schlägt der Stand der Technik verschiedene Maßnahmen vor, beispielsweise die Verwendung eines Ammoniaküberschusses oder eines Lösungsmittels, wenn damit zu rechnen ist, dass das Reaktionsgemisch durch das gebildete Wasser inhomogen wird (Houben-Weyl, Methoden der organischen Chemie, 4. Auflage, Georg Thieme Verlag Stuttgart, Bd XI/1, Seite 602 ff.).

DE 936211 beschreibt einen Flüssigphasenprozess zur Herstellung primärer aliphatischer Amine, Dabei wird der umzusetzende Aldehyd zunächst mit Ammoniak bei Temperaturen unterhalb von 0°C vermischt. Gegebenenfalls werden die Aldehyde mit einem niedrig siedenden Alkohol, beispielsweise Methanol, verdünnt. Anschließend wird diese Mischung bei erhöhter Temperatur und erhöhtem Druck in der Sumpf- oder Rieselfahrweise katalytisch hydriert, beispielsweise an einem Kobalt- oder Nickelkontakt.

Nach DE 199 35 448 A1 wird eine Mischung aus Methanol und Ammoniak mit Raney-Nickel versetzt und nach Beaufschlagung mit Wasserstoff auf Reaktionstemperatur aufgeheizt. Anschließend wird der Aldehyd zudosiert. Nach Beendigung der Reaktion wird der Ansatz entspannt, wobei Methanol und Ammoniak verdampfen. Anschließend wird das zurückgebliebene primäre aliphatische Amin weiter umgesetzt.

EP 0 628 535 A1 offenbart, den Aldehyd zunächst mit einem Verdünnungsmittel, beispielsweise mit Methanol oder Wasser bei höchstens 5°C abzumischen, um die Hemiacetal- oder Hydratbildung zu unterdrücken. In einem separaten Reaktionsgefäß werden Ammoniak, Wasserstoff und Nickelkatalysator bei erhöhter Temperatur und erhöhtem Druck vorgelegt, wobei im Reaktionsgefäß flüssiger Ammoniak vorliegt. Die gekühlte Mischung aus Aldehyd und Verdünnungsmittel wird unter Rühren in das Reaktionsgefäß gegeben. Nach beendeter Reaktion wird das Reaktionsgemisch über eine Fritte filtriert und das vom Katalysator befreite Rohgemisch aufgearbeitet. Das bekannte Verfahren lehrt die Verwendung eines hohen molaren Ammoniaküberschusses. Je Mol aliphatischen Aldehyd werden mindestens 15, vorzugsweise 20 bis 50 Mol, Ammoniak eingesetzt.

Nach dem Verfahren der US 2008/0227632 A1 erfolgt die reduktive Aminierung von Alkoholen, Aldehyden oder Ketonen in Gegenwart eines Katalysators, der neben Nickel, Kupfer und Chrom noch zusätzlich Zinn als aktives Metall enthält. Der Zusatz von Zinn erweist sich als vorteilhaft, um die Bildung von hydrierten Nebenprodukten zurückzudrängen. Das bekannte Verfahren eignet sich besonders zur Aminierung von einfach- oder mehrfach funktionellen Alkoholen, beispielsweise Ethylenglykol, Diethylenglykol oder Triethylenglykol.

Neben diesen einstufigen Prozessen behandelt der Stand der Technik auch zweistufige Verfahren, bei denen aus dem aliphatischen Aldehyd und überschüssigem Ammoniak zunächst in Gegenwart eines Iminierungskatalysators die Iminzwischenstufe gebildet wird, die anschließend mit Wasserstoff unter Verwendung eines Hydrierkatalysators in das primäre aliphatische Amin überführt wird. Gemäß EP 0 816 323 A2 wird bei dieser zweistufigen Arbeitsweise als Iminierungskatalysator ein Sulfonatgruppen enthaltendes Organopolysiloxan verwendet. Die dann folgende Hydrierung wird in Gegenwart von beispielsweise kobalt-, nickel- oder rutheniumhaltigen Katalysatoren durchgeführt.
Bei den bekannten einstufigen Verfahren arbeitet man entweder unter Verwendung eines Lösungs- oder Verdünnungsmittels oder man hat die Einsatzkomponenten unter Kühlung vorzumischen. Das Arbeiten mit einem Lösungs- oder Verdünnungsmittel erfordert die gezielte Abtrennung aus dem Reaktionsgemisch sowie zusätzliche logistische Maßnahmen, wie Rückführung und Lagerhaltung. Ebenfalls wird wertvolles Reaktorvolumen belegt und der Anlagendurchsatz geschmälert. Auch das Vormischen der Einsatzkomponenten unter Kühlung und das Einhalten niedriger Temperaturen in der Mischung vor dem Eintritt in den Reaktor bedeutet einen zusätzlichen apparativen Aufwand.

Aufgabe der vorliegenden Erfindung ist es daher, ein Verfahren zur Herstellung von primären aliphatischen Aminen bereitzustellen, das einen geringen technischen Aufwand benötigt und mit dem die gewünschten primären aliphatischen Amine mit hoher Selektivität erhalten werden. Insbesondere soll die Bildung von hochsiedenden Nebenprodukten möglichst zurückgedrängt werden.

Gegenstand der vorliegenden Efindung ist daher ein kontinuierliches Verfahren zur Herstellung von primären aliphatischen Aminen mit 9 bis 18 Kohlenstoffatomen durch Umsetzung von entsprechenden aliphatischen Aldehyden mit Ammoniak und Wasserstoff in Gegenwart eines Hydrierkatalysators in der Flüssigphase. Das Verfahren ist dadurch gekennzeichnet, dass die Umsetzung lösungsmittelfrei bei einem molaren Verhältnis von aliphatischem Aldehyd zu Ammoniak von mindestens 1 zu 30, bei einer Temperatur von 100 bis 200°C und bei einem Druck von 6 bis 11 MPa durchgeführt wird.

Überraschender Weise kann bei der lösungsmittelfreien Verfahrensführung die Bildung von selektivitätsmindernden Hochsiedern auf unter 10 Gew.-% im Reaktionsprodukt gedrückt werden, wenn bei der kontinuierlichen Prozessführung die Reaktionsbedingungen gezielt eingestellt werden. Auch ein separates Vormischen der Einsatzprodukte Ammoniak und aliphatischer Aldehyd unter Kühlung ist nicht erforderlich. Die Einsatzprodukte werden ohne weitere apparative Maßnahmen direkt aus ihren Vorlagen getrennt aber gleichzeitig in den Aminierungsreaktor gegeben.

Unter dem Begriff lösungsmittelfrei im Sinne der vorliegenden Erfindung versteht man, auf den aktiven Zusatz eines Lösungs- oder Verdünnungsmittels zu verzichten. Hingegen können geringe Mengen von Nebenbestandteilen in den Einsatzprodukten, beispielsweise herstellungsbedingte Alkoholreste in den Einsatzaldehyden, die Lösungsmittel- oder Verdünnungsmitteleigenschaften aufweisen, zugegen sein. Auch das während der Reaktion gebildete Wasser fällt nicht unter den Begriff Lösungsmittel oder Verdünnungsmittel, obwohl es gegenüber Ammoniak als Lösungsmittel wirkt.

Je Mol aliphatischen Aldehyd werden mindestens 30 Mol Ammoniak, vorzugsweise mindestens 35 Mol und insbesondere von 37 bis 45 Mol Ammoniak eingesetzt. Obwohl der hohe Ammoniaküberschuss zu einem Verdünnungseffekt führt und so der Bildung von Hochsiedern entgegenwirkt, sollte dennoch aufgrund des hohen Anteils der basisch reagierenden Verbindung die Aldehydkondensation in erheblichem Maße ablaufen, zumal nach der erfindungsgemäßen Arbeitsweise kein zugesetztes Lösungs- oder Verdünnungsmittel zugegen ist. Für das Erreichen wirtschaftlich vertretbarer Ausbeuten an primären aliphatischen Aminen ist es wesentlich, je Mol aliphatischen Aldehyd mindestens 30 Mol Ammoniak einzusetzen. Schon bei geringfügigem Unterschreiten dieses kritischen Wertes beobachtet man eine signifikante Zunahme an hochsiedenden Nebenprodukten.

Ohne auf reaktionsmechanistische Überlegungen eingehen zu wollen, kann vermutet werden, dass bei dem hohen Ammoniaküberschuss unter den einzustellenden Temperatur- und Druckbedingungen der aliphatische Aldehyd mit Ammoniak gesättigt ist und als ammoniakgesättigter Flüssigkeitsstrom durch den Aminierungsreaktor getrieben wird. Die vorliegende Flüssigkeit bewirkt vermutlich einen Wascheffekt auf die Oberfläche des Hydrierkatalysators und auf diese Weise können oberflächige Ablagerungen vom Hydrierkatalysator entfernt werden. Die Leistung des Hydrierkatalysators bleibt so über die Betriebsdauer erhalten und das zunächst gebildete Imin wird unmittelbar zu dem primären aliphatischen Amin hydriert. Bei abnehmender Hydrierleistung kann sich die Iminkonzentration im Reaktor erhöhen und durch Rückspaltung zu dem Aldehyd besteht dann die Gefahr der Bildung von hochsiedenden Aldehydkondensationsprodukten.

Die Umsetzung des aliphatischen Aldehyds mit Ammoniak in Gegenwart von Wasserstoff an dem Hydrierkatalysator erfolgt bei Temperaturen von 100 bis 200°C, vorzugsweise von 100 bis 170°C und insbesondere von 120 bis 150°C. Ab einer Temperatur von 170°C und besonders von 200°C beobachtet man eine Zunahme der Hochsiederbildung und damit eine Selektivitätsabnahme, während bei Temperaturen unterhalb von 100°C die Reaktionsgeschwindigkeit deutlich nachlässt. Der Reaktionsdruck beträgt von 6 bis 11 MPa, vorzugsweise von 7 bis 9 MPa, und er setzt sich im Wesentlichen aus den Partialdrücken von Wasserstoff und Ammoniak zusammen. Da die überkritischen Bedingungen von Ammoniak nicht erreicht werden, liegt neben gasförmigem Ammoniak im Aminierungsreaktor auch eine flüssige Phase aus ammoniakgesättigtem Einsatzaldehyd vor. Gleichzeitig kann der durch den Aminierungsreaktor getriebene Flüssigkeitsstrom als Spülmittel für den Hydrierkatalysator wirken und die Katalysatoroberfläche reinigen.

Als Hydrierkatalysatoren dienen übliche, bei der reduktiven Aminierung von Carbonylverbindungen verwendete Katalysatoren, die mindestens ein Metall der Nebengruppe 8 bis 11 des Periodensystems der Elemente enthalten, wie Nickel, Kobalt, Platin, Palladium, Eisen, Rhodium oder Kupfer. Besonders bevorzugt sind Nickel- oder Kobaltkatalysatoren. Neben trägerfreien Katalysatoren, wie Raney-Nickel oder Raney-Kobalt, können auch geträgerte Katalysatoren eingesetzt werden. Bei geträgerten Katalysatoren liegt im Allgemeinen das katalytisch aktive Metall in einer Menge von etwa 5 bis 70 Gew.-%, vorzugsweise etwa 10 bis etwa 65 Gew.-% und insbesondere etwa 20 bis 60 Gew.-%, jeweils bezogen auf das Gesamtgewicht des Hydrierkatalysators vor. Als Katalysatorträger eignen sich alle herkömmlichen Trägermaterialien, zum Beispiel Aluminiumoxid, Aluminiumoxidhydrate in ihren verschiedenen Erscheinungsformen, Siliciumdioxid, Polykieselsäuren (Kieselgele) einschließlich Kieselgur, Kieselxerogele, Magnesiumoxid, Zinkoxid, Zirconiumoxid und Aktivkohle. Neben den Hauptkomponenten katalytisch aktives Metall und Trägermaterial können die Hydrierkatalysatoren noch Zusatzstoffe in untergeordneten Mengen enthalten, die zum Beispiel der Verbesserung ihrer Hydrieraktivität und/oder ihrer Standzeit und/oder ihrer Selektivität dienen. Derartige Zusatzstoffe sind bekannt, zu ihnen gehören zum Beispiel die Oxide des Calciums, Bariums, Zinks, Aluminiums, Zirconiums und Chroms. Sie werden dem Hydrierkatalysator im Allgemeinen in einem Anteil von insgesamt 0,1 bis 30 Gew.-%, bezogen auf das Gesamtgewicht des Hydrierkatalysators, zugesetzt. Als bevorzugtes katalytisch aktives Metall hat sich Nickel erwiesen. Insbesondere sind Nickelkatalysatoren auf Kieselgur als Trägermaterial mit Chrom als Zusatzstoff für das erfindungsgemäße Aminierungsverfahren geeignet. Ganz besonders geeignet sind Nickelkatalysatoren, die 20 bis 60 Gew.-% Nickel, von 20 bis 70 Gew.-% Kieselgur und von 10 bis 20 Gew.-% Chrom enthalten, jeweils bezogen auf das Gesamtgewicht des Hydrierkatalysators und gegebenenfalls Füllstoffe mit Rest auf 100 Gew.-%.

Die aminierende Hydrierung wird in der Flüssigphase durchgeführt, beispielsweise an fest angeordneten Katalysatoren nach der Rieselfahrweise oder Sumpffahrweise sowie unter Rühren nach der Suspensionshydrierung. Neben Wasserstoff werden aliphatischer Aldehyd und Ammoniak dem Aminierungsreaktor getrennt aber gleichzeitig aus ihren Vorlagen zugeführt.

Vorzugsweise wird die kontinuierliche aminierende Hydrierung in flüssiger Phase in einem Rohrreaktor an fest angeordneten Hydrierkatalysatoren durchgeführt. Unter einem Rohrreaktor ist auch ein Bündel von mehreren eng parallel geschalteten Rohren zu verstehen. Die eingesetzten Rohrreaktoren können ebenfalls Füllkörper oder Einbauten enthalten, beispielsweise, Raschigringe, Sättel, Prallringe, Filterplatten oder Kolonnenböden, sowie gegebenenfalls Rührvorrichtungen. In einer besonders bevorzugten Ausgestaltung erfolgt die aminierende Hydrierung in einem Rohrreaktor jedoch ohne Einbauten mit geschüttetem Hydrierkatalysator.

Weniger bevorzugt ist das Arbeiten nach der Suspensionshydrierung, da der Betrieb von Rührvorrichtungen in dem unter Reaktionsdruck stehenden Aminierungsreaktor besondere Sicherheitsmaßnahmen erfordert. Auch ist die Abtrennung des in der Reaktionslösung suspendierten Hydrierkatalysators, beispielsweise über Filtriereinrichtungen, mit zusätzlichen Arbeitsschritten verbunden.

Bei der kontinuierlichen Fahrweise hat sich eine Katalysatorbelastung V/Vh mit aliphatischem Aldehyd, ausgedrückt in Durchsatzvolumen an aliphatischem Aldehyd pro Katalysatorvolumen und Zeit, von 0,02 - 0,50 h⁻¹, vorzugsweise 0,05 - 0,30 h⁻¹, als zweckmäßig erwiesen. Eine höhere Belastung des Hydrierkatalysators mit aliphatischem Aldehyd ist zu vermeiden, weil dann die aminierende Hydrierung nicht mehr vollständig erfolgt und aufgrund des hohen Aldehydrestgehaltes eine verstärkte Bildung von hochsiedenden Nebenprodukten beobachtet wird.

Bei zu geringen Durchsätzen pro Zeiteinheit wird die Anlagenkapazität nicht optimal ausgenutzt.

Die reduktive Aminierung erfolgt neben Ammoniak als Ausgangsverbindung vorzugsweise mit reinem Wasserstoff. Neben reinem Wasserstoff können jedoch auch Gemische verwendet werden, die freien Wasserstoff und daneben unter den Bedingungen der aminierenden Hydrierung inerte Bestandteile enthalten.

Die nach dem erfindungsgemäßen Verfahren umzusetzenden aliphatischen Aldehyde enthalten 9 bis 18 Kohlenstoffatome im Molekül, vorzugsweise 9 bis 15 und ganz bevorzugt 13 bis 15. Die Herkunft der aliphatischen Aldehyde ist nicht auf bestimme Herstellungsverfahren beschränkt.

Aufgrund ihrer leichten Zugänglichkeit werden durch Oxo-Synthese oder Hydroformylierung, d.h. durch Reaktion von C₈ - bis C₁₇-Olefinen mit Kohlenmonoxid und Wasserstoff gewonnene Aldehyde bevorzugt, wobei sowohl geradkettige als auch verzweigte Olefine, beispielsweise Olefinoligomere wie Tripropylen oder Tetrapropylen, als Ausgangsmaterialien für die Hydroformylierungsreaktion verwendet werden können. Als aliphatische Aldehyde eignen sich sowohl geradkettige n- als auch verzweigtkettige iso-Aldehyde entweder in reiner Form oder als Gemisch mit isomeren Aldehyden der gleichen Kohlenstoffzahl. Auch Gemische von aliphatischen Aldehyden mit unterschiedlicher Kohlenstoffzahl können verwendet werden. Besonders lassen sich nach der erfindungsgemäßen Arbeitsweise aliphatische Aldehyde mit 9 bis 15 Kohlenstoffatomen in die entsprechenden primären aliphatischen Amine überführen, beispielsweise Nonanal, Decanal, Undecanal, Dodecanal, Tridecanal, Tetradecanal oder Pentadecanal oder Mischungen davon. Die entsprechenden Aldehyde können dabei als geradkettige Verbindungen, als verzweigte Strukturisomere oder im Gemisch aus geradkettigen und verzweigten Strukturisomeren, auch mit unterschiedlicher Kohlenstoffzahl, eingesetzt werden. Das Gemisch aus geradkettigen n- und verzweigtkettigen iso-Aldehyden, die 13 und 15 Kohlenstoffatome im Molekül enthalten, ist für das erfindungsgemäße Aminierungsverfahren ganz besonders geeignet.

Das dem Aminierungsreaktor entnommene Reaktionsgemisch wird in einen Hochdruckabscheider geführt, wobei es zur Bildung einer gasförmigen und flüssigen Phase kommt. Die gasförmige Phase enthält im Wesentlichen Ammoniak und Wasserstoff sowie geringe Mengen an Reaktionswasser und wird abgeführt. Die erhaltene flüssige Phase wird über eine Standregelung auf Normaldruck entspannt und fließt in einen Vorlagebehälter. Bei dem Entspannungsvorgang entgast in der Flüssigphase gelöstes Ammoniak und gelöster Wasserstoff, die als Entspannungsabgas aus dem Vorlagebehälter abgeführt werden. Aus dem abgeführten Abgas des Hochdruckabscheiders und aus dem Entspannungsabgas kann Ammoniak zurückgewonnen und wieder in den reduktiven Aminierungsprozess zurückgefahren werden.

Aus der im Vorlagebehälter angesammelten flüssigen Phase werden anschließend Restmengen an Ammoniak und das Reaktionswasser entfernt. Das erhaltene primäre aliphatische Amin wird anschließend nach an sich bekannten Verfahren, beispielsweise durch Destillation, zu spezifikationsgerechter Ware aufgereinigt.

Durch das erfindungsgemäße Verfahren lassen sich aliphatische Aldehyde bei hohem Umsatz mit hoher Selektivität in die entsprechenden primären aliphatischen Amine überführen. Der Gehalt an hochsiedenden Nebenprodukten, gaschromatographisch ermittelt, in dem nach Abtrennung von Ammoniak und gebildetem Reaktionswasser erhaltenen Rohprodukt liegt dabei unter 10%.

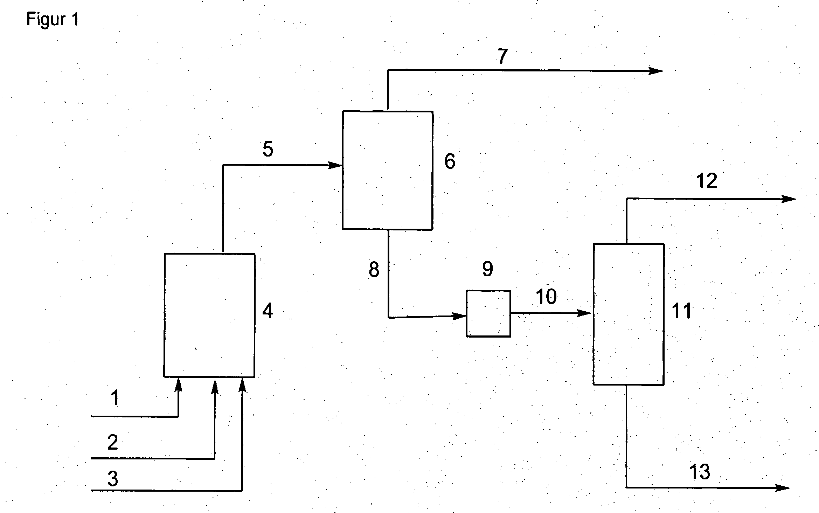

Das erfindungsgemäße Verfahren wird im Folgenden anhand des Prinzipschemas gemäß Figur 1 näher erläutert. Das erfindungsgemäße Verfahren ist aber nicht auf die in der Zeichnung dargestellte Ausführungsform beschränkt.

Über die Leitung (1) wird Ammoniak, über die Leitung (2) wird Wasserstoff und über die Leitung (3) aliphatischer Aldehyd in den mit dem Hydrierkatalysator gefüllten Aminierungsreaktor (4) kontinuierlich eingeleitet. Der Reaktoraustrag wird über die Leitung (5) abgezogen und in einen Hochdruckabscheider (6) geführt, in dem es zur Bildung einer gasförmigen und flüssigen Phase kommt. Die Gasphase aus dem Hochdruckabscheider (6) wird über die Leitung (7) abgeführt. Aus der abgeführten Gasphase, die im Wesentlichen aus Ammoniak und Wasserstoff besteht und Reaktionswasser in geringen Mengen enthält, wird Ammoniak zurückgewonnen und wieder über Leitung (1) in den Prozess zurückgeführt (nicht in Figur 1 gezeigt). Die im Hochdruckabscheider (6) erhaltene Flüssigphase wird über die Leitung (8) abgeführt, über eine Standregelung (9) auf Normaldruck entspannt und über Leitung (10) drucklos in den Vorlagebehälter (11) geführt. Die beim Entspannungsvorgang gebildeten gasförmigen Anteile, im Wesentlichen Restmengen aus gelöstem Ammoniak und Wasserstoff werden über die Leitung (12) ausgeschleust. Gegebenenfalls kann Ammoniak aus dem abgeführten Strom zurückgewonnen und zusammen mit frischem Ammoniak über Leitung (1) dem Aminierungsreaktor (4) wieder zugeführt werden (nicht in Figur 1 gezeigt). Über Leitung (13) wird die entgaste flüssige Phase abgeführt und anschließend nach an sich bekannten Verfahren destillativ aufgearbeitet (nicht in Figur 1 gezeigt).

Im Folgenden wird das erfindungsgemäße Verfahren anhand einiger Beispiele näher erläutert, es ist jedoch nicht auf die beschriebene Ausführungsform beschränkt.

### Versuchsaufbau

Die reduktive Aminierung erfolgte an einem kommerziellen, kieselgurgeträgerten Nickelkatalysator mit Chrom als Zusatzstoff in einem Rohrreaktor in der Sumpffahrweise. Das Katalysatorvolumen betrug 1,95 Liter. Als aliphatischer Aldehyd kam ein Gemisch aus geradkettigen n- und verzweigtkettigen iso-C₁₃- und C₁₅-Aldehyden zum Einsatz. Dieses Einsatzgemisch, Ammoniak und Wasserstoff wurden getrennt aber gleichzeitig am Sumpf des Rohrreaktors kontinuierlich zugeführt. Über den Kopf des Rohrreaktors entnahm man das Reaktionsprodukt und leitete es in einen Hochdruckabscheider. Die anfallende Flüssigkeit wurde über eine Standregelung auf Normaldruck entspannt und in eine drucklose Vorlage geführt. Das erhaltene organische Rohprodukt wurde anschließend gaschromatographisch analysiert.

Die Reaktionsbedingungen und die kontinuierliche Zufuhr der Einsatzstoffe wurden gemäß den Bedingungen der nachfolgenden Tabelle 1 eingestellt.

In Tabelle 1 ist ebenfalls die gaschromatographisch ermittelte Zusammensetzung des organischen Produkts, ammoniak- und wasserfrei, angegeben (in %).

Das für die Versuche verwendete aliphatische Aldehydgemisch wies folgende typische Zusammensetzung auf (gaschromatographisch ermittelt, Angaben in Prozent):

| | |
|---|---|
| C₁₂ und C₁₄-KW | 1,9 |
| n-/i-C₁₃-Aldehyd | 63,3 |
| n-/i-C₁₅-Aldehyd | 34,3 |
| Nachlauf C26-C30 | 0,5 |

**Tabelle 1: Reduktive Aminierung eines n-/iso-C₁₃C₁₅-Aldehydgemischs und gaschromatographische Analysen des erhaltenen rohen primären aliphatischen Amins in % (ammoniak- und wasserfrei)**

| Katalysatormenge ml/g | Beispiel 1 1950 ml/1095 g | Beispiel 2 1950 ml/1905 g | Beispiel 3 1950 ml/1905 g |
|---|---|---|---|
| Druck MPa | 8 | 8 | 8 |
| Katalysatortemperatur °C | 140 | 120 | 120 |
| Einsatz Wasserstoff l/h | 200 | 200 | 211 |
| Einsatz Aldehyd g/h | 414 | 103 | 300 |
| Einsatz Ammoniak g/h | 1500 | 1505 | 1500 |
| VNh bezogen auf Aldeyhd 1/h | 0,22 | 0,05 | 0,16 |
| | | | |
| Produktanalyse | | | |
| C₁₂ und C₁₄-KW | 1,7 | 1,7 | 1,8 |
| n/i-C₁₃-Amin | 61,0 | 60,4 | 61,9 |
| n/i-C₁₅-Amin | 32,3 | 33,8 | 33,5 |
| Nachlauf C26-C30 | 5,0 | 4,1 | 2,8 |

Wie die erfindungsgemäßen Beispiele belegen, kann der Gehalt an hochsiedenden Nebenprodukten in dem rohen primären aliphatischen Amin auf deutlich unter 10% gesenkt werden. Das erfindungsgemäße Verfahren gestattet die Gewinnung von primären aliphatischen Aminen mit hoher Selektivität bei geringem technischem Aufwand.

## Patentansprüche

1. Kontinuierliches Verfahren zur Herstellung von primären aliphatischen Aminen mit 9 bis 18 Kohlenstoffatomen durch Umsetzung von entsprechenden aliphatischen Aldehyden mit Ammoniak und Wasserstoff in Gegenwart eines Hydrierkatalysators in der Flüssigphase, **dadurch gekennzeichnet, dass** die Umsetzung lösungsmittelfrei bei einem molaren Verhältnis von aliphatischem Aldehyd zu Ammoniak von mindestens 1 zu 30, bei einer Temperatur von 100 bis 200°C und bei einem Druck von 6 bis 11 MPa durchgeführt wird.

2. Verfahren gemäß Anspruch 1, **dadurch gekennzeichnet, dass** das molare Verhältnis von aliphatischem Aldehyd zu Ammoniak von mindestens 1 zu 35, insbesondere von 37 bis 45, beträgt.

3. Verfahren gemäß Anspruch 1 oder 2, **dadurch gekennzeichnet, dass** die Umsetzung bei einer Temperatur von 100 bis 170°C erfolgt.

4. Verfahren gemäß Anspruch 3, **dadurch gekennzeichnet, dass** die Umsetzung bei einer Temperatur von 120 bis 150°C erfolgt.

5. Verfahren gemäß einem oder mehreren der Ansprüche 1 bis 4,
**dadurch gekennzeichnet, dass** die Umsetzung bei einem Druck von 7 bis 9 MPa erfolgt.

6. Verfahren gemäß einem oder mehreren der Ansprüche 1 bis 5,
**dadurch gekennzeichnet, dass** die umzusetzenden aliphatischen Aldehyde 9 bis 15, vorzugsweise 13 bis 15, Kohlenstoffatome im Molekül enthalten.

7. Verfahren gemäß einem oder mehreren der Ansprüche 1 bis 6,
**dadurch gekennzeichnet, dass** der Hydrierkatalysator mindestens Nickel, Kobalt, Platin, Palladium, Eisen, Rhodium oder Kupfer enthält.

8. Verfahren gemäß einem oder mehreren der Ansprüche 1 bis 7,
**dadurch gekennzeichnet, dass** der Hydrierkatalysator ein Trägermaterial enthält.

9. Verfahren gemäß einem oder mehreren der Ansprüche 1 bis 8,
**dadurch gekennzeichnet, dass** der Hydrierkatalysator als Zusatzstoffe Oxide des Catciums, Bariums, Zinks, Aluminiums, Zirconiums, Chroms oder Mischungen davon enthält.

10. Verfahren gemäß einem oder mehreren der Ansprüche 1 bis 9,
**dadurch gekennzeichnet, dass** man als Hydrierkatalysator einen Nickelkatalysator verwendet, der 20 bis 60 Gew.-% Nickel, von 20 bis 70 Gew.-% Kieselgur und von 10 bis 20 Gew.-% Chrom enthält, jeweils bezogen auf das Gesamtgewicht des Hydrierkatalysators und gegebenenfalls Füllstoffe mit Rest auf 100 Gew.-%.

11. Verfahren gemäß einem oder mehreren der Ansprüche 1 bis 10,
**dadurch gekennzeichnet, dass** die umzusetzenden aliphatischen Aldehyde als Gemisch von aliphatischen Aldehyden mit unterschiedlicher Kohlenstoffzahl verwendet werden.

12. Verfahren gemäß einem oder mehreren der Ansprüche 1 bis 11,
**dadurch gekennzeichnet, dass** ein Gemisch aus geradkettigen n- und verzweigtkettigen iso-Aldehyden, die 13 und 15 Kohlenstoffatome im Molekül enthalten, verwendet werden.

## Claims

1. Continuous process for producing primary aliphatic amines having 9 to 18 carbon atoms by reaction of corresponding aliphatic aldehydes with ammonia and hydrogen in the presence of a hydrogenation catalyst in the liquid phase, **characterized in that** the reaction is carried out solventlessly at a molar ratio of aliphatic aldehyde:ammonia of at least 1:30, at a temperature of 100 to 200°C and at a pressure of 6 to 11 MPa.

2. Process according to Claim 1, **characterized in that** the molar ratio of aliphatic aldehyde:ammonia is at least 1:35 and more particularly in the range from 37 to 45.

3. Process according to Claim 1 or 2, **characterized in that** the reaction is carried out at a temperature of 100 to 170°C.

4. Process according to Claim 3, **characterized in that** the reaction is carried out at a temperature of 120 to 150°C.

5. Process according to one or more of Claims 1 to 4, **characterized in that** the reaction is carried out at a pressure of 7 to 9 MPa.

6. Process according to one or more of Claims 1 to 5, **characterized in that** the aliphatic aldehydes to be reacted contain 9 to 15 and preferably 13 to 15 carbon atoms in the molecule.

7. Process according to one or more of Claims 1 to 6, **characterized in that** the hydrogenation catalyst contains at least nickel, cobalt, platinum, palladium, iron, rhodium or copper.

8. Process according to one or more of Claims 1 to 7, **characterized in that** the hydrogenation catalyst contains a support material.

9. Process according to one or more of Claims 1 to 8, **characterized in that** the hydrogenation catalyst contains oxides of calcium, of barium, of zinc, of aluminium, of zirconium, of chromium, or mixtures thereof, as added substances.

10. Process according to one or more of Claims 1 to 9, **characterized in that** the hydrogenation catalyst used is a nickel catalyst containing 20% to 60% by weight of nickel, from 20% to 70% by weight of kieselguhr and from 10% to 20% by weight of chromium, all based on the total weight of the hydrogenation catalyst and optionally fillers making up the balance to 100% by weight.

11. Process according to one or more of Claims 1 to 10, **characterized in that** the aliphatic aldehydes to be reacted are used as a mixture of aliphatic aldehydes having different numbers of carbon atoms.

12. Process according to one or more of Claims 1 to 11, **characterized in that** a mixture of straight-chain n- and branched-chain iso-aldehydes containing 13 and 15 carbon atoms in the molecule is used.

## Revendications

1. Procédé continu de fabrication d'amines aliphatiques primaires contenant 9 à 18 atomes de carbone par mise en réaction d'aldéhydes aliphatiques correspondants avec de l'ammoniac et de l'hydrogène en présence d'un catalyseur d'hydrogénation en phase liquide, **caractérisé en ce que** la réaction est réalisée sans solvant à un rapport molaire entre l'aldéhyde aliphatique et l'ammoniac d'au moins 1 sur 30, à une température de 100 à 200 °C et à une pression de 6 à 11 MPa.

2. Procédé selon la revendication 1, **caractérisé en ce que** le rapport molaire entre l'aldéhyde aliphatique et l'ammoniac est d'au moins 1 sur 35, notamment de 37 à 45.

3. Procédé selon la revendication 1 ou 2, **caractérisé en ce que** la réaction a lieu à une température de 100 à 170 °C.

4. Procédé selon la revendication 3, **caractérisé en ce que** la réaction a lieu à une température de 120 à 150 °C.

5. Procédé selon une ou plusieurs des revendications 1 à 4, **caractérisé en ce que** la réaction a lieu à une pression de 7 à 9 MPa.

6. Procédé selon une ou plusieurs des revendications 1 à 5, **caractérisé en ce que** les aldéhydes aliphatiques à mettre en réaction contiennent 9 à 15, de préférence 13 à 15, atomes de carbone par molécule.

7. Procédé selon une ou plusieurs des revendications 1 à 6, **caractérisé en ce que** le catalyseur d'hydrogénation contient au moins du nickel, du cobalt, du platine, du palladium, du fer, du rhodium ou du cuivre.

8. Procédé selon une ou plusieurs des revendications 1 à 7, **caractérisé en ce que** le catalyseur d'hydrogénation contient un matériau support.

9. Procédé selon une ou plusieurs des revendications 1 à 8, **caractérisé en ce que** le catalyseur d'hydrogénation contient en tant qu'additifs des oxydes de calcium, baryum, zinc, aluminium, zirconium, chrome ou leurs mélanges.

10. Procédé selon une ou plusieurs des revendications 1 à 9, **caractérisé en ce qu'**un catalyseur au nickel est utilisé en tant que catalyseur d'hydrogénation, qui contient 20 à 60 % en poids de nickel, de 20 à 70 % en poids de diatomée et de 10 à 20 % en poids de chrome, à chaque fois par rapport au poids total du catalyseur d'hydrogénation et éventuellement des charges, le reste permettant d'atteindre 100 % en poids.

11. Procédé selon une ou plusieurs des revendications 1 à 10, **caractérisé en ce que** les aldéhydes aliphatiques à mettre en réaction sont utilisés sous la forme d'un mélange d'aldéhydes aliphatiques présentant un nombre de carbones différent.

12. Procédé selon une ou plusieurs des revendications 1 à 11, **caractérisé en ce qu'**un mélange de n-aldéhydes linéaires et d'iso-aldéhydes ramifiés, qui contiennent 13 et 15 atomes de carbone par molécule, est utilisé.
